# EUROPÄISCHE PATENTSCHRIFT

(11) **EP 0 943 596 B1**
(45) Veröffentlichungstag und Bekanntmachung des Hinweises auf die Patenterteilung: **05.06.2002**
(21) Anmeldenummer: 99104350.6
(22) Anmeldetag: 04.03.1999
(51) Int. Cl.: C07C 29/149

(54) **Verfahren zur Herstellung von ungesättigten Fettalkoholen aus Lauricölen**
Process for the preparation of unsaturated fatty alcohols from lauric oils
Procédé pour la préparation d'alcools gras insaturés à partir d'huiles lauriques

(30) Priorität: 11.03.1998 DE 19810440
(43) Veröffentlichungstag der Anmeldung: 22.09.1999
(73) Patentinhaber: DHW Deutsche Hydrierwerke GmbH Rodleben, 06862 Rodleben (DE)
(72) Erfinder: Wieczorek, Frank, Dr.rer.nat., Dipl-Chem., 40670 Meerbusch (DE); Konetzke, Gerhard, Dr.rer.nat., Dipl-Chem., 06847 Dessau (DE); Seifert, Ekkehard, Dipl.Chem., 06862 Rosslau (DE)
(74) Vertreter: Tragsdorf, Bodo, Dipl.-Ing.

(56) Entgegenhaltungen:
- EP-A- 0 113 798
- DE-A- 4 129 622
- DE-A- 4 335 781
- DE-A- 4 425 180
- DE-A- 19 712 506

## Beschreibung

Die Erfindung betrifft ein Verfahren zur Herstellung von ungesättigten Fettalkoholen aus Lauricölen.

Ungesättigte Fettalkohole werden verglichen mit gesättigten Fettalkoholen nur in vergleichsweise geringen Mengen produziert, sind jedoch aufgrund ihrer speziellen Eigenschaften wichtige Zwischenprodukte für oleochemische Derivate oder Tenside, in Bereichen wie Kosmetik oder Pharmazie werden sie auch direkt eingesetzt. Derivate der ungesättigten Fettalkohole werden ebenfalls im Bereich Kosmetik und Pharmazie, überwiegend aber im technischen Bereich, z.B. als Hilfsmittel oder Schmierstoffbestandteil oder in Detergentien, eingesetzt. Ihre technische Darstellung erfolgt ausschließlich aus nativen Rohstoffen durch heterogen-katalytische Verfahren, wobei für bestimmte ungesättigte Endprodukte eine gezielte Rohstoffauswahl erfolgt, so daß im Rohstoff bereits wesentliche Teile des Kettenlängenspektrums vorgegeben sind. So waren langjährig Rohstoffe zur Herstellung ungesättigter Fettalkohole und sind es teilweise noch heute Talg, Schmalz, daraus durch verschiedene Anreicherungsverfahren hergestellte technische Oleine. Weitere Rohstoffe sind verschiedene höherjodzahlige Pflanzenöle wie Rapsöl, Sonnenblumenöl, Sojaöl, Palmöl und Baumwollsaatöl.

Seit einigen Jahren werden zur Herstellung ungesättigter Fettalkohole auch pflanzliche Rohstoffe, wie z.B. niederjodzahlige Öle, die sogenannten Laurics, eingesetzt. Diese weisen im Vergleich zu den tierischen Rohstoffen bessere sensorische Eigenschaften, wie Geruch und Farbe, auf und besitzen eine weit höhere Lagerstabilität, infolge eines geringeren Anteils an N-haltigen Nebenprodukten sowie weniger färbender Verunreinigungen in den gereinigten Rohstoffen. Bei den Lauricölen befinden sich die ungesättigten Bestandteile fast ausschließlich im Kettenlängenbereich C₁₈, wodurch deren Anreicherung möglich ist.
In der industriellen Praxis unterscheidet man bisher im wesentlichen drei Wege zur Herstellung von ungesättigten Fettalkoholen, die infolge der Verfügbarkeit unterschiedlicher Rohstoffe bzw. Zwischenprodukte nebeneinander Bedeutung erlangt haben.
1. Direkte Hydrierung von Triglyceriden zu ungesättigten Fettalkoholen und einigen Abbauprodukten des Glycerins, vor allem 1,2-Propandiol. Dieses Verfahren hat den wesentlichen Nachteil, daß das Glycerin zerstört wird und die gebildeten Abbauprodukte durch Waschung oder Extraktion aus dem Gemisch entfernt werden müssen. Außerdem entstehen weitere Kohlenwasserstoffe in größeren Mengen und müssen durch umständliche Fraktionierungen abgetrennt werden.
2. Spaltung der Öle oder Fette zu Fettsäuren und Glycerin, nach Abtrennung des Glycerins, Veresterung der Fettsäuren mit niederen Alkoholen wie Methanol oder Butanol zu dem entsprechenden Ester und Hydrierung dieser Ester zu ungesättigten Fettalkoholen unter Freiwerden des zur Veresterung eingesetzten Alkohols, der rezirkuliert werden kann. Dieser Weg ist relativ aufwendig durch die Vielzahl der Stufen sowie die destillativen Reinigungsnotwendigkeiten in diesen Stufen, die den Gesamtprozeß technisch und ökonomisch komplizieren.
3. Umesterung der Öle und Fette mit Methanol nach verschiedenen Verfahren mit homogenen bzw. heterogenen Katalysatoren zu Methylestern und Hydrierung dieser Methylester zu ungesättigten Fettalkoholen unter Rückgewinnung und Rezirkulierung des Methanols.

Während man nach der Variante 1 ungesättigte Fettalkohole mit einer Zusammensetzung erhält, die durch das Fettsäurespektrum des Öles vorbestimmt ist bzw. geringfügig durch Selektierung, partielle Hydrierung oder Kristallisation beeinflußt werden kann und umfangreichere Veränderungen nur in der Stufe der Alkohole möglich sind, kann man bei den Varianten 2 und 3 gezielte Veränderungen des Rohstoffs sowohl in den Vorstufen der Spaltfettsäuren, destillierten Fettsäuren, Methylester als auch in der Alkoholstufe vornehmen.
Der Einsatz von Lauricölen zur Herstellung von ungesättigten Fettalkoholen ist z.B. aus den Druckschriften DE 43 35 781 A1 und DE 44 25 180 C2 bekannt. Die Lauricöle weisen einen Schwerpunkt ihrer C-Kettenverteilung im Bereich C₁₂ bis C₁₄ auf und die ungesättigten Bestandteile befinden sich fast ausschließlich im Kettenlängenbereich C₁₈, wodurch ihre Anreicherung ermöglicht wird.
In der DE 43 35 781 A1 ist ein Verfahren zur Herstellung von ungesättigten Fettalkoholen mit einer lodzahl von 20 bis 110 aus Lauricölen beschrieben, wobei die in den Rohstoffen enthaltenen Triglyceride durch Druckspaltung in die Fettsäuren gespalten werden und ggf. mit Methanol verestert werden oder zu Fettsäuremethylestern umgeestert werden. Anschließend werden die Fettsäuren bzw. die Fettsäuremethylester zum Fettalkohol hydriert. Die Fettsäure, der Fettsäuremethylester und/oder das Produkt aus der Hydrierung werden abschließend fraktioniert. Vor der Fraktionierung wird die Iodzahl des zu fraktionierenden Produktes bestimmt und in Abhängigkeit von der ermittelten lodzahl und der gewünschten lodzahl wird bei der Fraktionierung eine bestimmte Vorlaufmenge entnommen, wodurch die lodzahl des Fettalkohols erhöht wird. Der Nachteil dieser Verfahrensweise besteht vor allem darin, daß aufwendige Fraktionierungen notwendig sind und verschiedene Kuppelfraktionen anfallen, für die nur eingeschränkte Verwertungsmöglichkeiten bestehen. Außerdem sind im C₁₈-Bereich die Anteile C₁₈:0 und C₁₈:1 nicht destillativ trennbar, sodaß nur eine bestimmte lodzahl einstellbar ist. Dieses Verfahren ist sehr kostenaufwendig und damit unwirtschaftlich.
Bei dem in der DE 44 25 180 C2 beschriebenen Verfahren werden die Lauricöle in Fettsäuren und Glycerin gespalten, die erhaltenen Spaltfettsäuren einer fraktionierten Kristallisation unterworfen, danach wird ggf. die Fraktion der ungesättigten Fettsäuren zu Methylestem verestert und abschließend zu ungesättigten Fettalkoholen mit einer lodzahl von 85 bis 100 hydriert. Derartige Fettalkohole weisen sehr gute Farb- und Geruchseigenschaften auf und zeichnen sich durch ein besonders vorteilhaftes Kälteverhalten aus.
Der bei dieser Verfahrensweise notwendige Schritt der Trennung von gesättigten und ungesättigten Fettsäuren ist vorzugsweise mittels einer sogenannten Umnetztrennung möglich, die aus der Trennung von Talgfettsäure in Stearin und Olein bekannt ist. Bei der Umnetztrennung der Talgfettsäure wird die technische Fettsäuremischung auf tiefe Temperaturen abgekühlt, wobei eine Kristallisation der Palmitin-/Stearinsäure in der flüssigen Ölsäure unter Bildung einer Dispersion stattfindet. Zum Abwaschen der Ölsäure von den Kristallen ist es erforderlich, der Dispersion eine wäßrige Netzmittellösung, ein Tensid, zuzusetzen.
Durch abschließendes Zentrifugieren der Emulsion/Dispersion in einem Separator wird diese in eine Ölsäurephase und eine Wasser/gesättigte Fettsäure-Dispersion getrennt. Die Fettsäure-Dispersion, die Palmitin-/Stearinsäure Wasserdispersion, muß nachfolgend auf etwa 50 bis 80 °C erwärmt werden, um die geschmolzene Palmitin/Stearinsäure von der wäßrigen Netzmittellösung abzutrennen, die im Kreislauf geführt wird.
Die Verfahrensstufe der Umnetztrennung zur fraktionierten Kristallisation der gemäß dem Verfahrensschritt a) erhaltenen Spalffettsäuren ist sehr zeitaufwendig und erfordert einen zusätzlichen apparativen Aufwand. Dadurch entstehen erhebliche Kostenbelastungen für das Endprodukt. Außerdem ist bei der Anwendung der Umnetztrennung von Spaltfettsäuren aus Lauricölen zu berücksichtigen, daß infolge der Zusammensetzung der Lauricöle in mehreren Verfahrensstufen ein großer Überschuß gesättigter Fettsäuren mit differenzierten Erstarrungsbereichen abgetrennt werden muß.
Der hohe Gehalt von 80 bis 90 % gesättigter C₁₂-C₁₈-Fettsäuren kann anstelle der Umnetztrennung auch durch fraktionierte Kristallisation mit Hilfe eines organischen Lösungsmittels abgetrennt werden. Die hierzu wie im Falle der Talgfettsäure üblichen Lösungsmittel, wie z.B. Methanol, Methanol/Wasser, Aceton oder Hexan, erfordern für den Kristallisationsvorgang Temperaturen von ≤ 0 °C bei hoher Verdünnung. Diese Verfahrensweise ist sehr aufwendig und erfordert einen hohen apparativen Aufwand zur Lösungsmittelhandhabung und -rückführung.

Der Erfindung lag die Aufgabe zugrunde, ein Verfahren zur Herstellung von ungesättigten Fettalkoholen aus Lauricölen zu schaffen, das sehr wirtschaftlich arbeitet und bei dem auf die Verfahrensschritte einer Umnetztrennung verzichtet werden kann, ohne nachteilige Auswirkungen auf die Qualität der Endprodukte.

Erfindungsgemäß wird die Aufgabe durch die im Anspruch 1 angegebenen Merkmale gelöst. Weitere Ausgestaltungsvarianten der Verfahrensweise sind in den Ansprüchen 2 bis 8 angegeben.
Die Lauricöle bilden die Hauptrohstoffbasis der nativen gesättigten C₁₂-C₁₆-Fettalkohle für die Detergentienindustrie. Sie ermöglichen eine optimale Verwertung der C₁₈-Anteile, die im Falle des Kokosöls ca. 11 % und im Falle des Palmkernöls ca. 20 % betragen. Diese Fraktion ist besonders für die Herstellung von ungesättigten Fettalkoholen geeignet. Die unterschiedliche Zusammensetzung der C₁₈-Fraktion ausgehend von Palmkern- und Kokosöl führt dazu, daß beide Öle prinzipiell geeignet, die Palmkernöl-C₁₈-Fraktion aber für die ungesättigten Fettalkohole optimal ist, da in ihr das Verhältnis C₁₈:0, C₁₈:1, C₁₈:2, C₁₈:3 besonders günstig sowohl für die selektive Hydrierung als auch im ungesättigten Fettalkohol für die Eigenschaften des Endproduktes ist.
Die Verfahrensweise zur Herstellung der ungesättigten Fettalkohole aus Lauricölen ist folgende:

### a) Umesterung

Die Umesterung der Laurics mit Methanol nach Entsäuerung des Rohstoffes ist ein großtechnisch beherrschter Prozeß, der neben Glyzerin die im Öl vorgebildete Kettenlängenverteilung der Methylester liefert.

### b) Fraktionierung

Zunächst wird der Vorlaufanteil C₆ bis C₁₀-ME und danach der C₁₂ - C₁₆-ME-Anteil für die gesättigten Fettalkohole abgetrennt. Aufgrund der Siedepunktdifferenzen fällt somit eine C₁₈-ME-Fraktion mit einem Gesamtanteil von C₁₈-Anteilen von ≥ 96 % an, wobei es ökonomisch von Vorteil ist, daß die kürzerkettigen Methylester quantitativ nicht der aufwendigen selektiven Hydrierung mit höheren Kosten unterworfen werden müssen.

### c) Winterisierung der C₁₈-ME-Fraktion

Die Winterisierung der C₁₈-ME-Fraktion erfolgt ohne Lösungsmittel durch Absenkung der Temperatur vorzugsweise in einem aber auch in mehreren Schritten auf Temperaturen von -10 °C bis +5 °C, Bildung einer Kristallfraktion in diesem Bereich, die vor allem die gesättigten Methylester enthält, in Größenordnung von 10 - 25 % und ihrer Abtrennung über ein geeignetes Filter. Je nach der Restiodzahl dieser ME-Kristallfraktion wird sie nachfolgend in Derivate verwandelt bzw. bevorzugt in gesättigte Fettalkohole überführt. Von allen untersuchten Kristallisationsprozessen der Laurics ist die von C₁₈-ME der am günstigsten verlaufende, da nach kurzer Verweilzeit ein Kristallbrei mit gut filtrierbaren festen Teilchen entsteht, der sich problemlos aufarbeiten läßt. Aus diesem Grund ist als Apparatur für diesen Schritt ein temperierbares gerührtes Kristallisationsgefäß, anschließend ein einfacher Filterprozeß, z.B. mit einem Platten- oder Bandfilter, ausreichend. Im Falle der Anwendung des bevorzugten Palmkernöls ist die Herstellung einer C₁₈-ME-Fraktion mit einem Gehalt von C₁₈-ME von 96,5 - 98,5 % durch Fraktionierung, sowie eine Abreicherung des Gehaltes an gesättigten Methylestern, vor allem von C₁₈:0-ME, von ca. 10 - 13 % auf 1 - 4 % im Filtrat der Winterisierungsstufe möglich.
Der nach der Winterisierungsstufe abgetrennte Kristallbrei mit dem Hauptanteil an C₁₈:0-ME beträgt etwa 15 - 35 % der Filtratmenge und besitzt eine lodzahl von 30 - 50. Eine weitere Trennung ist nicht nötig, da der bevorzugte Einsatz in der Hydrierung zu gesättigten Fettalkoholen und deren Abmischung mit hochungesättigten Fettalkoholen zu ungesättigten Fettalkoholen des lodzahlbereichs 40 - 60 erfolgt Wenn gewünscht, ist allerdings auch diese Fraktion der selektiven Hydrierung zugänglich und führt zu entsprechenden Gemischen ungesättigter mit gesättigten Fettalkoholen mit niedriger lodzahl.
Bei der Anwendung von Kokosöl erhält man in geringerer Ausbeute ebenfalls eine hochungesättigte C₁₈-ME-Fraktion, deren Abreicherung an C₁₈:0-ME ebenfalls mittels Winterisierung etwas ungünstiger verläuft und zu einem Restgehalt an C₁₈:0-ME im Filtrat von 3 - 5 % führt.

### d) selektive Hydrierung

Die ungesättigten ME-Fraktionen werden unter prinzipiell bekannten Bedingungen der selektiven Hydrierung zu ungesättigten Fettalkoholen unterworfen, so daß der maximale Gehalt ungesättigter Fettalkohole überwiegend in cis-Konfiguration mit nur geringen Anteilen von Nebenprodukten erhalten wird. Von den verschiedenen Katalysatorsystemen, die geeignet sind die Carboxylgruppenreduktion unter weitgehender Erhaltung olefinischer Doppelbindungen im Molekül zu vollziehen, werden hier heterogene Zinkoxid/Chromoxid-Systeme, teilweise promotiert mit aktivierenden Bestandteilen, wie Zr, Ba, Al, bevorzugt. Sie liefern ungesättigte Fettalkohole mit besonders niedrigen trans-Gehalten und geringen Anteilen von Stellungsisomeren. Die Bedingungen des kontinuierlich gestalteten Hydrierprozesses erfordern Drücke von 200 - 300 bar H₂ und hohe Temperaturen, wobei die Durchflußgeschwindigkeit des Esters über das Katalysatorbett bei verschiedenen Temperaturen im Bereich von ca. 0,1 - 0,4 v/vh variiert wird und dabei ein Umsatz von 99,0 - 99,8 % Ester erreicht werden kann. Zur Erhaltung der olefinischen Doppelbindungen ist an diesem Katalysatorsystem auch das Verhältnis Methylester zu H₂ von Bedeutung, wobei sich eine ausreichende Selektivität bei Anwendung von Molverhältnissen ME : H₂ von ≤ 1: 60 ergibt.

### e) destillative Reinigung

Die geringen Anteile gebildeter Nebenprodukte, wie gesättigte und ungesättigte Kohlenwasserstoffe sowie die durch Umesterung gebildeten Wachsester, lassen sich durch einfache Reinigungsdestillation als Vorlauf (1 - 2 %) bzw. Rückstand (1 - 5 %) nahezu quantitativ abtrennen.

Durch die Kombination der Verfahrensschritte a) bis e) in der angegebenen Reihenfolge wird ungesättigter Fettalkohol mit einer lodzahl zwischen 90 und 100 auf der Basis von Lauricölen nach einer sehr wirtschaftlichen Verfahrensweise erhalten. Die gewonnenen ungesättigten Fettalkohole stellen Gemische aus C₁₈-Alkoholen dar, wobei der Anteil der C₁₈:1-Fraktion, des Oleylalkohols, bei über 90 % liegt. Aus Palmkern-C₁₈-ME bzw. aus Kokos-C₁₈-ME werden nach der vorgeschlagenen Verfahrensweise rohe ungesättigte Fettalkohole mit einer lodzahl zwischen 90 und 100 in etwa folgender Zusammensetzung erhalten:

| | aus Palmkern C₁₈-ME | aus Kokos C₁₈-ME |
|---|---|---|
| | | |
| C₁₈:0 | 2 - 3 % | 3 - 5 % |
| cis C₁₈:1 | 87-93% | 80 - 90 % |
| trans C₁₈:1 | 3 - 6% | 4 - 8 % |
| C₁₈:2 | 2 - 3 % | 4 - 8 % |
| C₂₀:0 | 0 - 2 % | 0 - 2 % |
| C₂₀:1 | | |

Die Erstarrungspunkte dieser ungesättigten Fettalkohole liegen im Bereich zwischen 5 °C und 11 °C.
Die gewonnenen ungesättigten Fettalkohole mit einer lodzahl von 90 bis 100 können mit geeigneten anderen Fettalkoholen zu ungesättigten Fettalkoholen mit definiertem lodzahlbereich abgemischt werden, der in der Regel durch das Einsatzgebiet der ungesättigten Fettalkohole bestimmt ist. Ein hierfür geeigneter anderer Fettalkohol ist z.B. ein gesättigter Fettalkohol, der aus der im Verfahrensschritt b) anfallenden C₁₂ - C₁₆ Methylesterfraktion durch Hydrierung erhalten wird, wobei dieser gesättigte Fettalkohol durch weitere Fraktionierung in single-cuts zerlegt wird, die mit dem ungesättigten Fettalkohol gemischt werden.
Auch aus dem im Verfahrensschritt c) anfallenden Filterkuchen des Fettsäuremethylesters kann durch selektive Hydrierung ein teilungesättigter Fettalkohol erhalten werden, der als Abmischkomponente eingesetzt werden kann, um ungesättigte Fettalkohole mit niedrigen lodzahlen herzustellen.
Durch diese sinnvolle Verwertung der verfahrensgemäß anfallenden Nebenprodukte wird die Wirtschaftlichkeit des Verfahrens zusätzlich noch verbessert. Im Vergleich dazu ist eine Aufarbeitung der gemäß dem bekannten Stand der Technik als Nebenprodukt anfallenden Stearin-/Palmitinsäure zu einem abmischbaren Fettalkohol wesentlich aufwendiger.
Die nach dem erfindungsgemäßen Verfahren hergestellten ungesättigten Fettalkohole besitzen die vorteilhaften Eigenschaften der aus pflanzlichen Ölen herstellbaren Produkte, wie gute Sensorik, d.h. insbesondere eine gute Farb- und Geruchscharakteristik, gute Lagerstabilität und niedrige Erstarrungspunkte. Sie können deshalb vorteilhaft in typischen Anwendungsgebieten wie der Kosmetik oder in der Technik eingesetzt werden und zwar teilweise direkt als auch in Form ihrer Derivate, z.B. als Oxethylate, Sulfate und Ethersulfate, z.B. für Detergentien, technische Reinigungsmittel oder Hilfsmittel.

### Beispiel 1

### a) Umesterung von Palmkernöl (PKÖ)

In einem 4l-Glaskolben mit KPG-Rührer, Thermometer, Tropftrichter, Kühler, Vakuumvorstoß und Destillatvorlage wurden 2500 g eines marktüblichen PKÖ-Vollraffinats vorgelegt und 30 Min. bei 90 °C und ca. 35 mbar getrocknet, auf 60 °C gekühlt und erst 695 g Methanol, dann 55 g Na-methylatlösung 30%ig zugegeben. Der Kolbeninhalt wurde 1h bei 50 °C gerührt und anschließend in einen Scheidetrichter überführt. Nach ca. 45 min hatten sich 2662 g obere Phase (Rohester) und 560 g untere Phase (Glycerol, Methanol) abgeschieden.
Die obere Phase wurde 3mal mit 320 ml Trinkwasser bei ca. 50 °C gewaschen und anschließend 30 min bei 80 °C und ca. 35 mbar getrocknet. Es wurden 2395 g Palmkemöl-Methylester (PKÖ-ME) (roh) mit einer SZ (Säurezahl) = 0,06 erhalten. Das C-Kettenspektrum entsprach weitgehend dem PKÖ.

### b) Fraktionierung des PKÖ-ME

2300 g getrockneter PKÖ-ME wurden über eine mit Drahtwendeln gefüllte Kolonne bei einem Rücklaufverhältnis von 3 : 1 fraktioniert. Bei 0,5 mbar wurde im Siedebereich 155 170 °C folgende C₁₈-ME-Fraktion abgetrennt:
VZ (Verseifungszahl) = 184,7 IZ (lodzahl) = 83,3
C-Kettenverteilung: C₁₆ = 0,4 %, C₁₈:0 = 11,5 %, C₁₈:1 = 80,7 %, C₁₈:2 = 6,4 %, C₂₀ = 0,8 %.

### c) Fraktionierte Kristallisation (Winterisierung)

Zur Winterisierung wurde ein mantelgekühltes zylindrisches Glasgefäß mit einem Volumen von ca. 200 cm³ und einem langsam laufenden wandgängigen Rührer eingesetzt. Die Abfiltration des Feststoffanteils erfolgte in einer mantelgekühlten Filtemutsche mit einem mittelharten Filter, Filterfläche ca. 35 cm², die Filtertemperatur entsprach der Winterisierungstemperatur. Nach Einfüllen von 150 g C₁₈-ME bei ca. 30 °C wurde unter Rühren mit ca. 1 °C pro Minute abgekühlt und nach Erreichen der Winterisierungstemperatur 15 - 45 Minuten kristallisiert.

Durch fraktionierte Kristallisation bei +5 °C und einer Rührzeit von 15 min wurden aus der C₁₈-ME-Fraktion 13 % Filterkuchen mit einer IZ = 27,1 und folgender C-Kettenverteilung abgetrennt: C₁₆ = 0,5 %, C₁₈:0 = 69,4 %, C₁₈:1 = 27,4 %, C₁₈:2 = 1,6 %, C₂₀ = 1,1 %.

Der Filtratanteil betrug 87,7 % mit folgenden Kennzahlen: IZ = 88,5;
C-Kettenverteilung: C₁₆ = 0,3 %, C₁₈:0 = 3,2 %, C₁₈:1 = 88,5 %, C₁₈:2 = 7,2 %, C₂₀ = 0,8 %.

### d) Selektive Hydrierung

Der winterisierte C₁₈-ME wurde in einem kontinuierlich betriebenen Hochdruckreaktor an einem aktivierten ZnO Cr₂O₃-Katalysator selektiv hydriert. Die Hydrierbedingungen waren: 285 °C, 240 bar H₂-Druck, 0,1 v/vh C₁₈-ME und ein molares Verhältnis von C₁₈-ME zu H₂ von 1:100.

### e) Reinigungsdestillation

Der erhaltene, vom Methanol befreite Ablauf aus dem Hydrierreaktor wurde einer Reinigungsdestillation mit einem Druck von 0,5 mbar unterzogen, wobei 1,2 % Vorlauf (VL) abgetrennt wurden, im Destillationsrückstand verblieben 3,5 %. Der erhaltene C₁₈-UFA-HL (UFA = ungesättigter Fettalkohol, HL = Hauptlauf HL) wies nur einen äußerst schwachen Geruch auf und war farblos (APHA = 10). Der Carbonylgehalt betrug 53 mg/kg und stieg nach 1-monatiger Lagerung ohne N₂-Abdeckung nur um ca. 10 Einheiten an. Kennzahlen und C-Kettenspektrum des C₁₈-UFA-HL sind folgende:
SZ = 0,08, VZ = 0,5, IZ = 94,1, OHZ(Hydroxyl-Zahl) = 209,3, H₂O (K.F.) = 0,03 %, (K.F. = Anlaysenmethode nach Karl Fischer), Ep (Erstarrungspunkt) = 10,1 °C, KW-(Kohlenwasserstoff)-Gehalt <0,1 %.
C₁₆ = 0,2 %, C₁₈:0 = 4,3 %, cisC₁₈:1 = 86,5 %, transC₁₈:1 = 3,8 %, C₁₈:2 = 4,3 %, C₁₈total = 98,9 %, C₂₀ = 0.9 %.

Der erhaltene UFA-HL mit einer lodzahl von 94,1 wurde durch Zumischen von gesättigtem Fettalkohol (GFA) mit den C-Ketten C₁₂, C₁₄ und C₁₆ und einer Reinheit von jeweils mindestens 98 % - sogenannten single cuts - zu UFA mit einer niedrigen lodzahl abgemischt. Es wurden folgende UFA erhalten:
- - UFA 1:: IZ = 72,5, OHZ = 214,9, Ep = 19,8 °C aus 0,3 % C₁₂ + 2,7 % C₁₄ + 20,0 % C₁₆ + 77,0 % C₁₈-UFA-HL
- - UFA 2:: IZ = 84,0, OHZ = 213,0, Ep = 11,1 °C aus 1,0 % C₁₂ + 4,0 % C₁₄ + 5,7 % C₁₆ + 89,3 % C₁₈-UFA-HL

Die erhaltenen UFA weisen ausgezeichnete sensorische Eigenschaften auf, d.h. sie sind fast geruchlos und wasserhell.

### Beispiel 2

Es wurde analog wie im Beispiel 1 verfahren, wobei lediglich die Verfahrensparameter der Verfahrensstufe c), der Winterisierung, wie folgt geändert wurden:
Die Winterisierung der C₁₈-ME-Fraktion wurde bei einer Temperatur von - 2 °C und einer Rührzeit von 20 min durchgeführt.
Es wurden 21,3 % Filterkuchen abgetrennt, der eine IZ = 47,8 aufweist und folgende C-Kettenverteilung: C₁₆ = 0,1 %, C₁₈:0 = 47,5 %, C₁₈:1 = 48,1 %, C₁₈:2 = 3,7 %, C₂₀ = 0,6 %. Das Filtrat mit einer Ausbeute von 78,7 % war wie folgt charakterisiert:
VZ = 188 IZ = 92,6.
C-Kettenverteilung: C₁₆ = 0,5 %, C₁₈:0 = 1,8 %, C₁₈ :1 = 89,5 %, C₁₈ : 2 = 7,2 %, C₂₀ = 0,9 %.
Nach der Reinigungsdestillation wurden 1,5 % Vorlauf abgenommen und als Destillationsrückstand verblieben 3,9 %.
Der erhaltene C₁₈-UFA-HL ist wie folgt charakterisiert: SZ = 0,06, VZ = 0,4, IZ = 95,7, OHZ = 209,5, H₂O (K.F.) = 0,01 %, Ep = 5,2 °C, KW-Gehalt = < 0,1 %, APHA-Farbe = 8. C₁₆ = 0,3 %, C₁₈:0 = 2,6 %, cisC₁₈:1 = 87,9 %, transC₁₈:1 = 3,7 %, C₁₈:2 = 4,4 %, C₁₈total = 98,6 %, C₂₀ = 1,1 %.
Der C₁₈-UFA-HL hat eine lodzahl von 95,7 und nur einen Gehalt von C₁₈:0 von 2,6 % der den vergleichsweise niedrigen Ep von 5,2 °C mitbestimmt.
Damit ist dieser C₁₈-UFA besonders gut geeignet, um durch Mischung mit z.B. GFA single cuts einen UFA im IZ-Bereich 90 - 95 oder 80 - 85 herzustellen. Folgende UFA wurden durch Abmischen erhalten:
- - UFA 3:: IZ = 91,9, OHZ = 210,5, Ep = 6,6 °C durch Mischen von 4,0 % C₁₆ + 96,0 % C₁₈-UFA-HL
- - UFA 4:: IZ = 84,0, OHZ = 213,5, Ep = 9,2 °C durch Mischen von 1,0 % C₁₂ + 4,0 % C₁₄ + 7,2 % C₁₆ + 87,8 % C₁₈-UFA-HL.

Die UFA 3 und 4 weisen gute sensorische Eigenschaften auf, analog wie die gemäß Beispiel 1 hergestellten UFA 1 und 2.

Der in der Verfahrensstufe c), der Winterisierung, abgetrennte Filterkuchen (FK) enthält einen Anteil C₁₈:0 von 47,5 % und weist eine lodzahl IZ = 47,8 auf. Dieser Filterkuchen-ME wurde unter weitgehendem Erhalt der olefinischen Doppelbindung selektiv an einem ZnO/Cr₂O₃-Kontakt hydriert und anschließend einer Reinigungsdestillation analog wie im Verfahrensschritt e) von Beispiel 1 unterzogen, wobei 1,3 % VL abgetrennt wurden und 2,8 % Destillationsrückstand verblieben. Der erhaltende UFA-HL des Filterkuchens weist folgende Qualität auf:
SZ = 0,04, VZ = 0,2, IZ = 51,0, OHZ = 208,7, Ep = 38,8 °C, KW = 0,1 %,
C-Kettenverteilung: C₁₆ = 0,4 %, C₁₈ = 48,1 %, C₁₈:1 = 48,9 %, C₁₈:2 = 2,0 %, C₂₀ = 0,6 %.

Dieser erhaltene UFA weist im Vergleich zu marktüblichen UFA mit IZ von ca. 50 einen höheren Ep auf, ist aber dennoch gut zur Abmischung mit anderen Fettalkoholen zur Herstellung von UFA im IZ-Bereich von < 60 geeignet.
Durch Mischen einer Menge von
37 % dieses UFA-HL (FK), 35 % des gemäß Beispiel 2 hergestellten C₁₈-UFA-HL mit einer IZ = 95,7
sowie 0,5 % C₁₂, 2,5 % C₁₄ und 25 % C₁₆ gesättigter reiner Fettalkohole als sogenannte single cuts wurde folgender UFA 5 hergestellt, mit einer lodzahl von 52,4:
- - UFA 5:: IZ = 52,4, OHZ = 216, APHA = 10, Ep = 33,1 °C
C-Kettenverteilung: C₁₂ = 0,5 %, C₁₄ = 2,6 %, C₁₆ = 25,2 %, C₁₈ = 71,1 %, C₂₀ = 0,6 %.

### Beispiel 3

### a) Umesterung von Kokosöl (CNO)

Es wurde ein handelsübliches Kokosöl-Vollraffinat eingesetzt. Die Umesterung erfolgte unter analogen Bedingungen wie im Beispiel 1. Aus 2500 g CNO wurden 2380 g CNO-ME (roh) mit VZ = 256,8 und IZ = 8,0 erhalten. Das C-Kettenspektrum war nahezu identisch mit dem eingesetzten CNO.

### b) Fraktionierung des CNO-ME (roh)

2300 g CNO-ME (getrocknet) wurden über eine mit Drahtwendeln gefüllte Kolonne bei einem Rücklaufverhältnis von 4 : 1 fraktioniert. Aufgrund des vergleichsweise zur PKO-Basis niedrigeren C₁₈-Anteils im CNO-ME wurden bei 0,8 mbar im Siedebereich 152 - 172 °C 11,7 % C₁₈-ME-Fraktion folgender Qualität gewonnen:
SZ = 0,08, VZ = 188,6, IZ = 76,9
C₁₆ = 1,2 %, C₁₈:0 = 19,5 %, C₁₈:1 = 66,9 %, C₁₈:2 = 11,8 %, C₂₀ = 0,6 %.

### c) Fraktionierte Kristallisation des C₁₈-ME (Winterisierung)

Die Winterisierung erfolgte in analoger Weise wie im Beispiel 1 angegeben. Durch fraktionierte Kristallisation bei +5 °C und einer Rührzeit von 30 min wurden aus der C₁₈-ME-Fraktion 24,1 % Filterkuchen (FK) mit einer IZ = 33,2 und folgender Kettenverteilung:
C₁₆ = 2,1 %, C₁₈:0 = 67,6 %, C₁₈ :1 = 21,6 %, C₁₈:2 = 8,3 %, C₂₀ = 0,4 % sowie
75,9 % Filtrat mit einer IZ = 92,5 und folgender Kettenverteilung:
C₁₆ = 1,0 %, C₁₈:0 = 4,2 %, C₁₈ :1 = 81,3 %, C₁₈:2 = 12,9 %, C₁₈total = 98,8 %, C₂₀ = 0,6 % erhalten.

### d) Selektive Hydrierung

Die selektiven Hydrierbedingungen des C18-ME-Filtrats entsprachen den im Beispiel 1 genannten.

### e) Reinigungsdestillation

Bei der Reinigungsdestillation des methanolfreien Rohalkohols bei 0,6 mbar wurden 1,6 % VL abgenommen, im Destillationsrückstand verblieben 3,1 %.
Der so gewonnene C₁₈-UFA-HL ergab folgende Qualität:
SZ = 0,05, VZ = 0,5, IZ = 95,1, OHZ = 209,5, APHA = 15, Ep = 10,2 °C, CO-Gehalt = 82 ppm, H₂O (K.F.) = 0,03 %, KW-Gehalt = 0,11 %
C₁₆ = 0,4 %, C₁₈:0 = 3,9 %, cisC₁₈:1= 84,7 %, transC₁₈:1 = 3,2 %, C₁₈:2 = 6,7 %, C₁₈total = 98,5 %, C₂₀ = 1,1 %.

Der erhaltene C₁₈-UFA-HL wurde mit sogenannten single cuts gesättigter reiner Fettalkohole zu einem marktüblichen UFA abgemischt. Zur Mischung wurden eingesetzt:
0,4 % C₁₂+ 2,8 % C₁₄ + 20,3 % C₁₆ + 76,5 % C₁₈-UFA-HL.
Erhalten wurde ein UFA im IZ-Bereich 70 - 75 mit folgender Qualität:
IZ = 72,8, OHZ = 215,1, Ep = 18,9 °C
C₁₂ = 0,4 %, C₁₄ = 2,8 %, C₁₆ = 20,5 %, C₁₈ = 75,5 %, C₂₀ = 0,8 %.

## Patentansprüche

1. Verfahren zur Herstellung von ungesättigten Fettalkoholen aus Lauricölen, **dadurch gekennzeichnet, daß** man
a) die entsäuerten Lauricöle mit Methanol zum Gesamtfettsäuremethylestergemisch umestert,
b) das Fettsäuremethylestergemisch durch Fraktionierung in eine für gesättigte Fettalkohole bestimmte Methylesterfraktion C₁₂-C₁₆ und in eine den nahezu vollständigen Anteil der ungesättigten Methylester (ME) enthaltende C₁₈-ME-Fraktion trennt,
c) die erhaltene C₁₈-ME-Fraktion einer lösungsmittelfreien fraktionierten Kristallisation, einer Winterisierung, unterwirft, um den gesättigten C₁₈-ME-Anteil abzutrennen,
d) die winterisierte ungesättigte C₁₈-ME-Fraktion der selektiven Hydrierung zu ungesättigten Fettalkoholen mit einer Jodzahl zwischen 90 und 100 unterwirft und
e) die rohen ungesättigten gemäß Verfahrensschritt d) erhaltenen Fettalkohole einer destillativen Reinigung unterzieht.

2. Verfahren nach Anspruch 1, **dadurch gekennzeichnet, daß** man den gereinigten ungesättigten Fettalkohol mit geeigneten anderen Fettalkoholen zu ungesättigten Fettalkoholen des gewünschten Jodzahlbereiches abmischt.

3. Verfahren nach einem der Ansprüche 1 oder 2, **dadurch gekennzeichnet, daß** man die gemäß Verfahrensschritt b) erhaltene C₁₂-C₁₆ Methylesterfraktion zu gesättigten Fettalkoholen hydriert, diese durch Fraktionierung in single-cuts zerlegt und als andere Fettalkohole zur Abmischung mit den ungesättigten Fettalkoholen einsetzt.

4. Verfahren nach einem der Ansprüche 1 bis 3, **dadurch gekennzeichnet, daß** man den gemäß Verfahrensschritt c) anfallenden Filterkuchen des Fettsäuremethylesters selektiv hydriert und die teilungesättigten Fettalkohole als andere Fettalkohole zur Abmischung mit den ungesättigten Fettalkoholen einsetzt.

5. Verfahren nach einem der Ansprüche 1 bis 4, **dadurch gekennzeichnet, daß** man die fraktionierte Kristallisation unter Bedingungen durchführt, bei denen ein Filtratanteil von 70 bis 80 % und ein C₁₈:0 mit einem Gehalt von 1,5 bis 3,5 % erreicht wird.

6. Verfahren nach einem der Ansprüche 1 bis 5, **dadurch gekennzeichnet, daß** man die fraktionierte Kristallisation bei einer Temperatur von +5 °C bis -10 °C durchführt.

7. Verfahren nach einem der Ansprüche 1 bis 6, **dadurch gekennzeichnet, daß** man zur selektiven Hydrierung einen Fixbettkatalysator bestehend aus einem aktivierten thermisch vorbehandelten ZnO/Cr₂O₃-System einsetzt.

8. Verfahren nach einem der Ansprüche 1 bis 7, **dadurch gekennzeichnet, daß** man als Ausgangsrohstoff raffiniertes Palmkernöl einsetzt.

9. Verfahren nach einem der Ansprüche 1 bis 7, **dadurch gekennzeichnet, daß** man als Ausgangsrohstoff raffiniertes Kokosöl einsetzt.

## Claims

1. A process for producing unsaturated fatty alcohols from lauric oils, **characterized in that**
(a) the deacidified lauric oils are converted by ester interchange with methanol into the total fatty acid methyl ester mixture;
(b) the fatty acid methyl ester mixture is separated by fractionation into a C₁₂-C₁₆-methyl ester fraction intended for saturated fatty alcohols, and a C₁₈ -ME-fraction containing nearly the complete proportion of the unsaturated methyl esters (ME);
(c) the C₁₈-ME fraction so obtained is subjected to a solvent-free fractionated crystallization, i.e., to a winterization for separating the saturated C₁₈-ME proportion;
(d) the winterized unsaturated C₁₈-ME fraction is subjected to selective hydrogenation to unsaturated fatty alcohols with an iodine number between 90 and 100; and
(e) the crude unsaturated fatty alcohols obtained according to process step (d) are subjected to distillatory purification.

2. The process according to claim 1, **characterized in that** the purified unsaturated fatty alcohol is mixed with suitable other fatty alcohols to obtain unsaturated fatty alcohols with the desired iodine number range.

3. The process according to any one of claims 1 and 2, **characterized in that** the C₁₂-C₁₆ methyl ester fraction obtained according to process step (b) is hydrogenated to saturated fatty alcohols, the latter are split by fractionation into single cuts, and used as other fatty alcohols for mixing with the unsaturated fatty alcohols.

4. The process according to any one of claims 1 to 3, **characterized in that** the filter cake of the fatty acid methyl ester collected according to process step (c) is selectively hydrogenated, and that the partially saturated fatty alcohols are used as other fatty alcohols for mixing with the unsaturated fatty alcohols.

5. The process according to any one of claims 1 to 4, **characterized in that** the fractionated crystallization is carried out under conditions leading to a filtrate component of 70 to 80% and a C₁₈:O with a content of 1.5 to 3.5%.

6. The process according to any one of claims 1 to 5, **characterized in that** the fractionated crystallization is carried out at a temperature of +5 °C to -10 °C.

7. The process according to any one of claims 1 to 6, **characterized in that** a fixed-bed catalyst consisting of an activated, thermally pretreated ZnO/Cr₂O₃-system is employed for the selective hydrogenation.

8. The process according to any one of claims 1 to 7, **characterized in that** refined palm kernel oil is used as the starting raw material.

9. The process according to any one of claims 1 to 7, **characterized in that** refined coconut oil is used as the starting raw material.

## Revendications

1. Procédé de fabrication d'alcools gras insaturés à partir d'huiles lauriques, **caractérisé**
a) en ce que les huiles lauriques désacidifiées sont estérifiées avec le méthanol pour former un mélange d'esters méthyliques d'acides gras globaux,
b) en ce que le mélange d'esters méthyliques d'acides gras est séparé par fractionnement en une fraction d'esters méthyliques C₁₂-C₁₆ déterminée pour les alcools gras saturés et une fraction C₁₈-ME contenant la portion presque complète des esters méthyliques insaturés (ME),
c) en ce que la fraction C₁₈-ME obtenue est soumise à une cristallisation fractionnée exempte de solvant, à un hivernage, pour séparer la portion saturée C₁₈-ME,
d) en ce que la fraction insaturée C₁₈-ME, ayant subi l'hivernage, est soumise à une hydrogénation sélective pour former des alcools gras insaturés ayant un indice d'iode compris entre 90 et 100, et
e) en ce que les alcools gras bruts insaturés, obtenus conformément à l'étape de procédé d), sont soumis à une purification par distillation.

2. Procédé selon la revendication 1, **caractérisé en ce que** l'on mélange l'alcool gras insaturé purifié à des alcools gras appropriés supplémentaires pour former des alcools gras insaturés du domaine d'indice d'iode souhaité.

3. Procédé selon l'une quelconque des revendications 1 ou 2, **caractérisé en ce que** l'on procède à l'hydrogénation de la fraction d'esters méthyliques C₁₂-C₁₆, obtenue conformément à l'étape de procédé b), pour former des alcools gras saturés, à la décomposition de cette dernière **en ce que** l'on appelle des "single-cuts", et **en ce que** l'on utilise cette fraction en tant qu'alcools gras supplémentaires en vue du mélange avec les alcools gras insaturés.

4. Procédé selon l'une quelconque des revendications 1 à 3, **caractérisé en ce que** l'on procède à l'hydrogénation sélective du gâteau de filtre de l'ester méthylique d'acide gras se formant conformément à l'étape de procédé c) et **en ce que** l'on utilise les alcools gras partiellement insaturés en tant qu'alcools gras supplémentaires en vue du mélange aux alcools gras instaurés.

5. Procédé selon l'une quelconque des revendications 1 à 4, **caractérisé en ce que** l'on effectue la cristallisation fractionnée dans des conditions dans lesquelles l'on parvient à une portion de filtrat de 70 à 80% et à un C₁₈:0 ayant une teneur de 1,5 à 3,5%.

6. Procédé selon l'une quelconque des revendications 1 à 5, **caractérisé en ce que** l'on effectue la cristallisation fractionnée à une température de +5°C à -10°C.

7. Procédé selon l'une quelconque des revendications 1 à 6, **caractérisé en ce que** l'on utilise, en vue de l'hydrogénation sélective, un catalyseur à lit fixe se composant d'un système ZnO/Cr₂O₃ activé, ayant subi un traitement thermique préalable.

8. Procédé selon l'une quelconque des revendications 1 à 7, **caractérisé en ce que** l'on utilise, en tant que matière première brute de départ, l'huile de palmiste raffinée.

9. Procédé selon l'une quelconque des revendications 1 à 7, **caractérisé en ce que** l'on utilise, en tant que matière première brute de départ, l'huile de noix de coco raffinée.
